# EUROPÄISCHE PATENTANMELDUNG

(11) **EP 4 509 592 A2**
(43) Veröffentlichungstag der Anmeldung: **19.02.2025**
(21) Anmeldenummer: 24181557.0
(22) Anmeldetag: 12.06.2024
(51) Int. Cl.: C12M 1/107, B30B 9/14, C12M 1/00, C12P 5/02, C02F 11/121

(54) **SEPARATOR, SYSTEM UND VERFAHREN ZUR ERZEUGUNG VON BIOGAS**

(30) Priorität: 28.07.2023 DE 102023120154
(71) Anmelder: Fritz Paulmichl GmbH, 88299 Leutkirch im Allgäu (DE)
(72) Erfinder: Paulmichl, Frank, 88317 Aichstetten (DE)
(74) Vertreter: Otten, Roth, Dobler & Partner mbB Patentanwälte

(57) **Zusammenfassung**

Es wird ein Separator (1) zur Fest-Flüssig-Trennung eines dem Separator (1) zuführbaren Substrats in einem angetriebenen Trennbetrieb des Separators (1) vorgeschlagen, wobei im Trennbetrieb ein Teil des Substrats den Separator (1) in eine Strömungsrichtung durchströmt und das Substrat in eine Flüssigphase und in eine Festphase trennbar ist, die aus dem Separator (1) abführbar sind, wobei der Separator (1) ein Gehäuse (2), einen Einlass (3) für das Substrat, einen Flüssigphasen-Auslass (4), einen Auslass (5) für die Festphase, einen Fördermechanismus (6) und/oder einen Verdichtermechanismus (7) für das Substrat umfasst. Erfindungsgemäß ist der Auslass (5) gasdicht mit einem in Strömungsrichtung stromabwärts angeordneten Behälter verbindbar, wobei im verbundenen Zustand des Auslasses (5) die Festphase selbstfördernd in den Behälter gelangt.

## Beschreibung

### Stand der Technik

In der Trenntechnik zur Trennung eines Mehrkomponenten-Gemisches in unterschiedliche Phasen sind Separatoren bekannt, welche nach dem Trennprinzip mittels Zentrifugalkräften arbeiten.

Separatoren für Gemische nutzen unter Rotation einer angetriebenen Welle des Separators die Zentrifugalwirkung auf die von der Welle bewegten Komponenten des Gemisches zur Erhöhen der auf die Komponenten des Gemisches wirkenden Beschleunigung. Zur Fest-Flüssig-Trennung mit Separatoren müssen die zu trennenden Komponenten des Gemisches unterschiedliche Dichten aufweisen.

Separatoren kommen in unterschiedlichen verfahrenstechnischen Einsatzgebieten zur Anwendung, zum Beispiel bei kontinuierlichen Trennverfahren im landwirtschaftlichen Bereich zur Auftrennung von flüssiger bzw. fließfähiger Biomasse wie Substraten. Zum Beispiel dient die Auftrennung der Substrate zur Bereitstellung von eingedickten organischen Anteilen des Substrats, die biologisch abbaubare Biomasse enthalten und als Wertstoff nutzbar sind, zum Beispiel zur Gewinnung von Biogas als nichtfossiler Energieträger.

Zur Steigerung der Effektivität der Biogaserzeugung bzw. zur Reduzierung eines notwendigen Volumens eines Biogasbehälters werden Separatoren zum Eindicken des Substrats wie zum Beispiel Gülle oder Gärsubstrat wie z. B. Maische eingesetzt. Beim Eindicken von Biomasse-Substraten ist die Konsistenz der eingedickten Festphase eine verfahrenstechnische Herausforderung zum Beispiel was die Förderung und die Handhabung der eingedickten Festphase und die Abstimmung des Trennprozesses bezüglich unterschiedlicher Verfahrensparameter angeht.

### Aufgabe und Vorteile der Erfindung

Aufgabe der vorliegenden Erfindung ist es, im Bereich der Separation von Biomasse-Substraten eine verbesserte Prozessführung zu ermöglichen. Insbesondere soll bei Biogas-Anlagen, bei denen eine vorgeschaltete Fest-Flüssigtrennung des Substrates mit einem Separator stattfindet, eine zuverlässige und effektive Eindickung des Wertstoffes und Beschickung des Biogasbehälters möglich sein. Darüber hinaus sollen Prozess-Störungen im Umfeld der Separation ausgeschlossen oder minimiert werden.

Diese Aufgabe wird durch die unabhängigen Ansprüche gelöst. Die abhängigen Ansprüche zeigen vorteilhafte und zweckmäßige Weiterbildungen der Erfindung auf.

Die Erfindung geht aus von einem Separator zur Fest-Flüssig-Trennung eines dem Separator zuführbaren Substrats in einem angetriebenen Trennbetrieb des Separators, wobei im Trennbetrieb ein Teil des Substrats den Separator in eine Strömungsrichtung durchströmt und das Substrat in eine Flüssigphase und in eine Festphase trennbar ist, die aus dem Separator abführbar sind, wobei der Separator ein Gehäuse, einen Einlass für das Substrat, einen Flüssigphasen-Auslass, einen Auslass für die Festphase, einen Fördermechanismus und/oder einen Verdichtermechanismus für das Substrat umfasst. Beispielsweise weist der Separator eine Beschickungs-Seite auf, welche dem Einlass für das Substrat zugeordnet ist und eine Auslassseite, die dem Auslass zugeordnet ist. Eine einlassseitige Beschickung des Separators mit Substrat erfolgt zum Beispiel mit einer Verdränger-Pumpe bzw. einer Druck-Pumpe wie z. B. einer Exzenterschneckenpumpe.

Beispielsweise kann der Einlass für das Substrat so ausgebildet sein, dass die Strömungsrichtung des zugeführten Substrats einlassseitig von oben z. B. senkrecht ist, zum Beispiel das Substrat senkrecht von oben in den Separator zuführbar ist. Alternativ ist der Einlass für das Substrat unten am Separator vorhanden, wobei die Strömungsrichtung des zugeführten Substrats senkrecht von unten nach oben verläuft.

Die Festphase ist durch den Separator eingedicktes Substrat, also Substrat, von dem Teile der Flüssigphase separiert bzw. abgetrennt wurden.

Zum Beispiel ist der Separator als Rotations-Separator in der Art z. B. einer Zentrifuge ausgebildet mit einer antreibbaren rotierenden Achse bzw. Welle. Der Antrieb der Achse bzw. Welle erfolgt zum Beispiel über einen Antriebsmotor wie z. B. über einen Elektromotor.

Zum Beispiel ist unter Substrat alles zu verstehen, was Biomasse enthält und z. B. im landwirtschaftlichen Bereich zu Gas oder Dünger verarbeitet werden kann.

Zum Beispiel umfasst der Auslass für die Festphase bzw. des Fest- oder Dickstoffes eine Öffnung bzw. eine Auslassöffnung des Separators. Beispielseise liegt die Öffnung bzw. die Auslassöffnung in Förder- bzw. Längsrichtung des Separators betrachtet in einem Bereich des Separators, der gegenüberliegt zu einem Bereich des Separators, in welchem des Einlass des Separators vorhanden ist. Zum Beispiel erstreckt sich die Achse über die wesentliche Länge des Separators, z. B. durchgehend über einen Abstand zwischen zwei endseitigen Drehlagerstellen des Separators zur Drehlagerung der Achse.

In der Regel ist die Strömungsrichtung bzw. die Förderrichtung des Separators horizontal ausgerichtet, z. B. bei horizontal ausgerichteter Achse.

Beispielsweise liegt ein Aspekt der Erfindung darin, dass der Auslass gasdicht mit einem in Strömungsrichtung stromabwärts angeordneten Behälter verbindbar ist, wobei im verbundenen Zustand des Auslasses die Festphase selbstfördernd in den Behälter gelangt.

Zum Beispiel umfasst die gasdichte Verbindung zwischen dem Auslass und dem Behälter eine lösbare Verbindung. Alternativ umfasst die Verbindung zwischen dem Auslass und dem Behälter eine nicht lösbare Verbindung wie z. B. eine Schweißverbindung.

Zum Beispiel umfasst die gasdichte Verbindung zwischen dem Auslass und dem Behälter eine Pressverbindung, eine Schraubverbindung oder eine Flanschverbindung.

Zum Beispiel ist die gasdichte Verbindung zwischen dem Auslass und dem Behälter nicht manuell einrichtbar, sondern mit einem Werkzeug einrichtbar und wieder lösbar. Zum Beispiel ist die gasdichte Verbindung zwischen dem Auslass und dem Behälter manuell und/oder werkzeuglos einrichtbar und wieder lösbar. Zum Beispiel ist die gasdichte Verbindung zwischen dem Auslass und dem Behälter durch eine Schnellverbindung einrichtbar und wieder lösbar.

Beispielsweise umfasst die Verbindung einen Rohrabschnitt, wie zum Beispiel ein Tauchrohr, der durch eine Öffnung in einer Wand des Behälters reicht. Im Bereich des Durchtritts ist der Rohrabschnitt außen abgedichtet zur Öffnung in der Wand.

Zum Beispiel weist der Auslass eine Dichteinrichtung auf, zur gasdichten Verbindung mit dem in Strömungsrichtung stromabwärts angeordneten Behälter. Zum Beispiel ist die Dichteinrichtung als eine bekannte und bewährte Dichteinrichtung ausgebildet. Beispielseise umfasst die Dichteinrichtung eine Flach-Ringdichtung mit einem elastisch verformbaren Dichtelement zwischen benachbarten Dichtflächen. Zum Beispiel ist eine Dichtfläche dem Auslass zugehörig und eine weitere Dichtfläche ist dem Behälter zugehörig.

Beispielsweise weist der Behälter eine Seitenwand oder eine Oberseite auf, mit einer Öffnung, die z. B. seitlich oder nach oben am Behälter offen ist. Die Öffnung ist offen zum Inneren des Behälters bzw. grenzt an das Innenvolumen des Behälters an. Die Öffnung des Behälters ist mit einer Öffnung des Auslasses leitend verbunden, so dass bei montiertem Separator die Festphase bzw. der Feststoff bzw. der Dickstoff aus dem Separator über den Auslass in das Innere des Behälters gelangt.

Zum Beispiel ist die Verbindung zwischen dem Auslass und dem Behälter durch eine Flanschverbindung realisierbar. Zum Beispiel weist der Separator im Bereich des Auslasses für die Festphase einen entsprechend ausgebildeten Flanschring auf. Damit ist z. B. eine Flanschverbindung mit einem zwischen den Flanschen gepresst vorhandenem Dichtring einrichtbar. Die z. B. beiden Flansche sind mit Schraubmitteln verbindbar, zum Beispiel mit Schrauben, die durch Bohrungen in die aneinander liegenden Flansche gesteckt werden.

Zum Beispiel ist der Behälter ein Faul-, Biogas-, Gär-, Schlamm-, Dickstoff- oder Vorlagebehälter.

Beispielsweise ist unter einem Substrat ein Gemisch zu verstehen, das Biomasse enthält bzw. das Substrat ist selbst Biomasse. Zum Beispiel ist Substrat ein Stoffgemisch, das z. B. in landwirtschaftlichen oder anderen Einrichtungen der Landwirtschaft bzw. bei landwirtschaftlichen Betrieben anfällt und zur Biogas-Erzeugung herangezogen werden kann.

Beispielsweise enthält Substrat Ausscheidungen von Tieren wie Rinder- oder Schweine-Gülle oder das Substrat enthält pflanzliche Biomasse wie z. B. Maische oder gehäkselte Maispflanzen.

Beispielsweise dient Gülle, Gärsubstrat oder Maische als Substrat, also Biomasse, die durch Gärprozesse u.a. in Biogas umgesetzt wird. Zum Beispiel kommt Rinder- oder Schweinegülle oder andere Gülle aus der Tierhaltung als Substrat in Frage. Auch im Obst-, Gemüse- und/oder im Weinanbau fallen Stoffe an, z. B. Substrate, die als Gärsubstrat bezeichnet werden können, und als Substrat zur Biogaserzeugung dienen können. In der Regel müssen die Ausgangsstoffe, welche die betreffende Biomasse zur Erzeugung des Biogases enthalten, durch einen Separator eingedickt werden, zum Beispiel zur Steigerung der biochemischen und verfahrenstechnischen und damit zur Steigerung der wirtschaftlichen Effektivität der Biogasanlage.

Als Substrat dient z. B. organisches Material bzw. Biomasse aus der Landwirtschaft, das vergleichsweise gut biochemisch abbaubar ist, zum Beispiel durch Mikroorganismen. Bei der Nutzung der Biomasse für die Biogasherstellung mit einer Biogasanlage wird die Biomasse in einem Biogas-Behälter unter anaeroben Bedingungen von anaerob lebenden Mikroorganismen in Biogas und andere Stoffe verstoffwechselt bzw. biochemisch umgesetzt werden. Dabei bilden sich unter biochemischen Faulvorgängen gasförmige Kohlenwasserstoffe wie z. B. Methan (CH₄), die als gasförmiger Energieträger bzw. als brennbare Gase zum Beispiel mit hoch effizienten Verbrennungsmotoren optimal für die Energiegewinnung genutzt werden können. Zum Beispiel dient das aus der FeststoffPhase des Substrats erzeugte Biogas als Energiequelle für den Betrieb von Gas- bzw. Verbrennungsmotoren. Diese Motoren werden demgemäß zur Erzeugung von elektrischer Energie und/oder Wärmeenergie in Form von Abwärme eingesetzt. Diese Art der Energiegewinnung aus Biomasse dient der Reduzierung des Bedarfs an fossilen Energieträgern wie Öl und Gas und damit dem Ziel der Klimaneutralität zum Beispiel mit Blick auf die Vermeidung von ungewollten Folgen der menschgemachten Klimaveränderung.

Beispielsweise ist unter einer selbstfördernden Verbindung eine durch die Schwerkraft bedingte und/oder unterstützte Bewegung der eingedickten Festphase zu verstehen. Zum Beispiel spielen dabei konstruktive Eigenschaften im Bereich des Auslasses zur Förderung der Festphase vom Separator zum Behälter eine Rolle. Zum Beispiel spielt eine räumliche Ausrichtung einer an den Auslass sich anschließenden Förderstrecke für die Festphase in Richtung des Behälters eine Rolle, wie z. B. ein Neigungswinkel eines geraden und/oder gekrümmten Auslassrohres, das sich an den Auslass anschließt und die eingedickte Festphase in Strömungsrichtung vom Auslass in das Innere des Behälters führt. Beispielseise ist das Innere des Auslassrohres mit einer glatten Oberfläche bzw. mit vergleichsweise sehr geringer OberflächenRauheit bzw. Rauigkeit versehen, womit die noch meist feuchte eingedickte Festphase leichtgängig nach unten in das Innere des Behälters rutsch, z. B. ohne weitere notwendige Fördermaßnahme zum Weitertransport der Festphase.

Es ist auch denkbar durch z. B. Aufbringen einer Vibration- oder Schlagbewegung auf das Auslassrohr von außen die Bewegung der Festphase vom Auslass des Separators weg in Richtung des Inneren des Behälters zu unterstützen.

Zum Beispiel wird mit dem vorgeschlagenen Separator einerseits vermieden, durch die Gasdichtigkeit der Verbindung des Separators zum Behälter, dass im Bereich zwischen dem Separator und dem Behälter Biogas nach außen entweichen kann, was nachteilig wäre. Außerdem wird vermieden, dass Luft-Sauerstoff ins Innere des Behälters gelangt.

Andererseits kann durch die selbstfördernde Verbindung auf Fördermaßnahmen zur Förderung der aus dem Separator ankommender Festphase in den Behälter verzichtet werden. Dies ist z. B. im Hinblick auf andernfalls störanfällige Fördermaßnahmen zum Weitertransport der Festphase vorteilhaft. Beispielsweise lassen sich dahingehende Störungen im Betrieb des Separators vermeiden.

Beispielsweise ist der Separator als Gülle- und/oder Biogas-Gärsubstrat-Separator ausgebildet, umfassend ein Gehäuse, einen Gülle- und/oder Biogas-Gärsubstrat-Einlass, einen Güllefeststoff- und/oder Biogas-Gärsubstratfeststoff-Auslass, einen Flüssigphasen-Auslass, einen Gülle- und/oder Biogas-Gärsubstrat-Fördermechanismus und einen Verdichtermechanismus, wobei der Güllefeststoff- und/oder Biogas-Gärsubstratfeststoff-Auslass gasdicht mit einem in Strömungsrichtung stromabwärts angeordneten Gärbehälter verbindbar ist, wobei im verbundenen Zustand des Auslasses der Güllefeststoff und/oder der Biogas-Gärsubstratfeststoff selbstfördernd in den Gärbehälter gelangt.

Damit können in der Landwirtschaft bei Betrieben mit Tierhaltung, bei denen Gülle anfällt, und/oder bei Betrieben, bei denen gärfähige Biomasse wie Gärsubstrat anfällt, und das Gülle- bzw. Gärgut-Substrat einer Eindickung unterzogen werden muss, Biogas-Anlagen unter verbesserten verfahrenstechnischen Bedingungen betrieben werden.

Zum Beispiel umfasst der Fördermechanismus eine Förderschnecke, welche auf einer doppelt gelagerten Achse angeordnet ist. Damit wird ein robuster Förder- und Eindickprozess ermöglicht. Mit einer Förderschnecke wird eine mechanisch bewährte und effektive Eindickung ermöglicht. Für einen Antrieb der rotierbar bzw. drehbar gelagerten Achse ist ein motorischer Antrieb des Separators vorgesehen. Zum Beispiel umfasst der Antrieb einen Elektromotor. Zum Beispiel ist der Antrieb ausgebildet, unterschiedliche Drehzahlen der Achse zu ermöglichen.

Der Fördermechanismus dient zum Beispiel zum kontinuierlichen Fördern der Gülle- und/oder des Biogas-Gärsubstrats in Strömungsrichtung.

Beispielsweise ist die Achse über zwei Lagerstellen, die über die Länge der Achse voneinander beabstandet sind, gelagert. Zum Beispiel ist die Achse im Bereich beider Längs-Enden der Achse bzw. im Bereich beider Achsenden gelagert. Die doppelte Lagerung ermöglicht zum Beispiel eine sichere und stabile Lagerung der Achse.

Zum Beispiel weist die Förderschnecke ein Schneckengewinde auf. Die Achse bildet z. B. die Schneckenwelle des Fördermechanismus. Zum Beispiel ist die Förderschnecke als einfach gewendeltes Schneckengewinde ausgebildet. Zum Beispiel ist die Förderschnecke als mehrfach gewendeltes Schneckengewinde ausgebildet. Zum Beispiel ist die Förderschnecke als einfach oder als mehrfach gewendeltes Schneckengewinde ausgebildet. Zum Beispiel erstreckt sich die Förderschnecke über eine wesentliche Länge der Achse bzw. der Schneckenwelle. Zum Beispiel erstreckt sich die Förderschnecke mit dem Schneckengewinde von einem einlassseitigen Endbereich der Achse bis zu einem Bereich, in dem eine Membrane des Verdichtermechanismus vorhanden ist. Beispielsweise ist ein Ende der Förderschnecke bzw. des Schneckengewindes etwas beabstandet von der Membrane. Zum Beispiel reicht die Achse durch die Membrane durch bis zu einem Ende der Achse, die im Bereich des Separators auslassseitig gelagert ist.

Beispielsweise umfasst der Verdichtermechanismus eine Membrane aus einem elastischen Material. Beispielsweise umfasst die Membrane ein Gummi-Material, ein Elastomer-Material oder ein anderes gummiartiges Material. Beispielsweise ist die Membrane eine abhängig vom Umgebungsdruck auf beiden Seiten der Membrane sich öffnende und schließende Membrane. Die Membrane öffnet und schließt druckabhängig bzw. abhängig von einem resultierenden Druck auf einer Seite der Membrane. In der Regel ist zeitweise der Druck auf der Seite des Fördermechanismus größer als auf der Seite des Auslasses, weil dort die Festphase nicht ansteht, sondern weiter in den Behälter gelangt.

Zum Beispiel trennt die Membrane in Förderrichtung bzw. in Strömungsrichtung das Innere des Separators in voneinander durch die Membrane getrennte Teilbereiche. In einem einlassseitigen Teilbereich erstreckt sich der Fördermechanismus. In einem auslassseitigen Teilbereich befindet sich der Auslass für die Festphase.

Bei Druckanstieg auf einer Seite der Membran durch die Eindickung der Festphase aber verformt sich bzw. öffnet sich die Membrane zeitweise und die Festphase kann in Förderrichtung die Membrane passieren.

Im nicht verformten Zustand der Membrane zum Beispiel, trennt die Membrane das Innere des Separators zwischen einem Eindickbereich, umfassend die Achse mit der Förderschnecke, und dem Bereich des Auslasses, in dem ein weiterer Abschnitt der Achse ohne eine Förder- bzw. Eindickschnecke vorhanden ist. Aus diesem Bereich gelangt die Festphase selbstfördernd in den Behälter.

Zum Beispiel umfasst der Bereich mit dem Fördermechanismus auch einen eindickend wirkenden Bereich z. B. umfassend ein Sieb oder ein Lochblech wie zum Beispiel eine Siebtrommel oder ein Spaltsieb. Durch Öffnungen im Sieb oder Lochblech tritt abgetrennte Flüssigphase über den Flüssigphasen-Auslass aus dem Separator nach außen und fließt ab.

Nicht ausgeschlossen ist die Zugabe von Flockungshilfsmittel in das zufließende Substrat, was zu einer besseren Fest-Flüssig-Trennung führt.

Die Membrane kann auf unterschiedliche Weise ausgebildet sein. Zum Beispiel ist die Membrane in der Art einer Festphasen-Schleuse ausgebildet. Zum Beispiel gibt die Membrane ein Widerstand vor, der überwunden werden muss, damit eine Weiterförderung der gebildeten Festphase erfolgt. Zum Beispiel mit Erreichen eines vorgebbaren Feststoffgehalts der Festphase verformt sich ein elastischer Bereich der Membrane oder verformen sich mehrere elastische Bereiche der Membrane derart, z. B. Flügelabschnitte, dass sich die Flügelabschnitte aufweiten und eine Durchlassöffnung in der Membrane zum Durchlass von Festphase öffnet. Zum Beispiel verbiegt sich ein Flügelabschnitt dabei entlang eines Filmscharniers der Membrane. Zum Beispiel besteht das Filmscharnier aus dem Membranmaterial.

Mit dem Öffnen der Abschnitte der Membrane gelangt eingedickte Festphase in Förderrichtung vom Bereich der Fördereinrichtung mit der Förderschnecke in den Bereich des Auslasses für die Festphase. Zum Beispiel ab einem erreichten Eindickgrad der Festphase passiert die Festphase die Membrane. Zum Beispiel passiert die Festphase die Membrane abhängig von deren elastischem Aufweit-Verhalten für die in Förderrichtung einseitig an der Membrane drückend anstehende Festphase. Zum Beispiel passiert die Festphase eine durch Ausweichen der Flügelabschnitte zeitweise vorhanden Durchlassöffnung in der Membrane. Die Festphase gelangt hinter der Membrane in den Bereich des Auslasses für den Feststoff.

Nach dem Passieren einer gewissen Menge der Festphase verringert sich der an der Membrane anstehende Druck und die elastischen Abschnitte bzw. Flügelabschnitte weichen selbsttätig wieder zurück bis in die von der Membrane vorgegeben Ebene bei unbelasteter Membrane. Die Durchlassöffnung verschließt sich dabei wieder bis zum nächsten Anstieg des Druckwertes auf einen Gasdruck- bzw. Druck-Durchlasswert. Der Vorgang wiederholt sich immer wieder im Trennbetrieb. Festphase wird quasikontinuierlich durch den Separator in Richtung des Auslasses gefördert.

Zum Beispiel kann das elastische Material und/oder eine Dicke der Membrane abgestimmt sein auf die Verdichterfunktion der Membrane. Zum Beispiel kann im Trennbetrieb des Separators bei gleicher sonstiger Ausgestaltung des Separators, durch Variation der Art und Gestaltung der Membrane z. B. ein Eindickgrad der Festphase vorgegeben werden. Beispielsweise kann die Anzahl und/oder die Art der in der Membrane ausgebildeten Flügelabschnitte bezüglich eines elastischen Verhaltens der Flügelabschnitte vorgegeben sein. Mit der Vorgabe des elastischen Verhaltens der Membrane ist ein Widerstand der Membrane gegen ein Durchströmen von Festphase vorgebbar. Zum Beispiel ist ein Widerstandswert der Membrane abhängig vom Substrat bzw. der eingedickten Festphase vorgebbar.

Zum Beispiel ist die Membrane scheibenförmig. Zum Beispiel ist die Membrane so gebildet, dass sie radial außen an das Innere des Gehäuses zumindest nahezu dicht anschließt, zum Beispiel die Membrane entlang eines Außenrandes der Membrane mit dem Gehäuse verbunden ist. Zum Beispiel ist die Membrane ringscheiben-förmig, bei hohlzylindrischem Gehäuse. Die Membrane weist entsprechend eine zentrische kreisförmige Öffnung auf. Diese Öffnung ist im Betrieb des Separators dauerhaft von der durchtretenden Achse z. B. der angetriebenen Welle verschlossen.

Zum Beispiel tritt die Achse zentrisch durch die Membrane, die hierfür die entsprechende Öffnung entsprechend einem Außendurchmesser der Achse aufweist, die durch die Öffnung durchtritt. Bei einer in der Regel zylindrischen Achse weist die Membrane eine kreisförmige zentrische Öffnung zum gleitenden Durchgreifen der Achse auf. Beispielsweise reicht ein Rand der Öffnung bis an die Außenseite bzw. eine Außenzylinder-Mantelfläche der Achse heran.

Beispielsweise umfasst die Membrane in einer Fläche quer zur Förderrichtung bzw. Strömungsrichtung, segmentartig ausgebildete Flügelabschnitte in einem geschlossenen System. Die Membrane mit ihren Flügelabschnitten bildet in einem unbelasteten Zustand z. B. ohne in Förderrichtung geförderter Feststoffphase eine axial geschlossene Scheibe im Inneren des Gehäuses des Separators. Beispielsweise bildet die Membrane, wenn die Flügelabschnitte nicht elastisch verformt sind, sondern in der Ebene der Scheibenform der Membrane sich befinden, eine Abtrennung eines Eindickbereichs im Innern des Separators gegenüber einem Auslassbereich des Separators, von dem aus die Feststoffphase in den Behälter selbstfördernd gelangt.

Im Trennbetrieb des Separators wird durch die Förderschnecke der Dickstoff bzw. die Festphase durch die Membrane gedrückt, wobei die Flügelabschnitte sich in Förderrichtung elastisch verbiegen. Damit bildet und vergrößert sich eine Öffnung in der Membran zeitweise und ermöglicht einen Durchlass für den Feststoff in Förderrichtung.

Beispielsweise weist die Membrane mehrere Einschnitte oder linienförmige schmale Öffnungen auf. Beispielsweise weist die Membrane mehrere linienförmige und voneinander beabstandete Einschnitte auf. Zum Beispiel verlaufen die mehreren Einschnitte, zum Beispiel drei bis acht Einschnitte, von der zentrischen Öffnung radial nach außen. Beispielsweise reichen die Einschnitte in der Membran in radialer Richtung zur Längsachse der Achse nach außen bis zu einem Punkt, der radial beabstandet ist zu einem radial äußeren, umfänglich verlaufenden Rand der Membrane. Im Bereich des Punktes, an dem der jeweilige Einschnitt endet, kann z. B. zur Vermeidung von Kerbwirkung ein lochartiger Ausschnitt in der Membran ausgebildet sein, in welchen der Einschnitt mündet und dort endet.

Beispielsweise begrenzen zwei benachbarte Einschnitte jeweils dazwischen einen Flügelabschnitt. Bei z. B. acht Einschnitten in der Membrane sind acht Flügelabschnitte ausgebildet, die zum Beispiel identisch geformt sind.

Die mehreren Flügelabschnitte bilden mit ihren jeweiligen radial innenliegenden Rändern die zentrische Öffnung in der Membrane, zum Durchgreifen der Achse.

Zum Beispiel bildet die Membrane im nicht verformten bzw. im unbelasteten Zustand bei nicht ausgelenkten Flügelabschnitten eine festphasen- und/oder gasdichte Barriere z. B. in und entgegen der Förder- bzw. Strömungsrichtung im Separator bzw. in einem dazugehörigen System.

Zum Beispiel gibt der Auslass eine im Wesentlichen vertikal nach unten weisende Auslassrichtung vor, so dass die Weiterförderung der Festphase ausschließlich durch Gravitationskraft erfolgt.

Beispielsweise mündet der Auslass in eine Auslassleitung wie z. B. eine Rohrleitung bzw. einen Rohrabschnitt. Beispielsweise weist der Auslass und ein daran anschließender Abschnitt der Auslassleitung den gleichen Innendurchmesser auf. Beispielsweise weist die Auslassleitung über ihre gesamte Länge einen gleichbleibenden Innendurchmesser auf.

Die Auslassleitung wie z. B. der Rohrabschnitt überbrückt mit vorgebbarer Ausrichtung bzw. ausgerichtet einen Abstand zwischen dem Auslass und einer Stelle im Inneren des Behälters. Beispielsweise weist die Auslassleitung bzw. der Rohrabschnitt ein nach unten offenes Ende mit einer Leitungs- bzw. Rohröffnung auf.

Beispielsweise ist der Rohrabschnitt gerade ausgebildet wie ein gerades Rohr.

Beispielsweise umfasst der Rohrabschnitt einen an den Auslass anschließenden Winkel-Rohrabschnitt und einen daran anschließenden geraden Rohrabschnitt, der z. B. als Tauchrohr ausgebildet ist. Der Rohrabschnitt schleißt an den Auslass bzw. eine dazugehörige Auslassöffnung ans, zum Beispiel ist am Auslass angeflanscht. Beispielsweise endet ein unteres Ende des Rohrabschnitts bzw. des Tauchrohres unterhalb einer Behälter-Füllhöhe bis zu welcher der Behälter mit Festphase gefüllt ist.

Zum Beispiel reicht der Rohrabschnitt mit einem unteren Ende bzw. mit einem unteren Rand des Rohrabschnittes bis in einen Bereich einer maximalen Füllhöhe bzw. einer Standard-Füllhöhe des Behälters.

Zum Beispiel reicht der Rohrabschnitt mit einem unteren Ende bzw. mit einem unteren Rand des Rohrabschnittes bis in einen Bereich, der in senkrechter Richtung bzw. vertikal betrachtet unterhalb einer maximalen Füllhöhe bzw. unterhalb einer Standard-Füllhöhe des Behälters liegt.

Die Füllhöhe bezieht sich auf eine Höhe bzw. ein Füllniveau des mit Festphase befüllten Behälters zum Beispiel im Betriebszustand zur Erzeugung von Biogas im Behälter. Zum Beispiel kann kein Biogas über das unten offene Ende des Rohrabschnitts nach oben in den Separator gelangen. Zum Beispiel ist der Separator außerhalb des Behälters etwas oberhalb der maximalen Füllhöhe bzw. etwas oberhalb einer Standard-Füllhöhe für die Festphase im Behälter positioniert. Ohne eine auf die Festphase aufgezwungene Strömung kann gemäß dem Prinzip der kommunizierenden Röhren keine Festphase aus dem Inneren des Behälters über den Auslass in das Innere des Separators zurückfließen.

Zum Beispiel weist der Güllefeststoff- und/oder Biogas-Gärsubstratfeststoff-Auslass eine im Wesentlichen vertikal nach unten weisende Ausrichtung auf, so dass der Güllefeststoff und/oder Biogas-Gärsubstratfeststoff ausschließlich durch Gravitationskraft weitergefördert wird.

Beispielsweise weist der Separator für eine Unterstützung der Weiterförderung der eingedickten Festphase wie Güllefeststoff oder Gärsubstratfeststoff, einen Spülanschluss und/oder eine Spülleitung auf. Die Weiterförderung dient dem Transport der Festphase aus dem Separator hinaus und weiter in den Behälter hinein.

Beispielsweise dient der Spülanschluss und/oder die Spülleitung zur Zuführung eines Spülmediums wie z. B. einer Spülflüssigkeit, z. B. Wasser, das Substrat selbst oder einer bei der Separation vom Substrat anfallenden Flüssigkeit, wie z. B. dem Presswasser.

Beispielsweise ist die Spülleitung so positioniert, dass die Flüssigkeit die Festphase unmittelbar nach dem Pressvorgang mitnimmt. Beispielsweise kann die Flüssigkeit in Förderrichtung betrachtet im Bereich hinter der Membrane oder im Bereich des Auslasses eingeleitet werden. Die Spülleitung ist z. B. so positioniert, dass die Flüssigkeit über die Spülleitung in Förder- bzw. Strömungsrichtung des Separators gespült wird. Die Strömungsrichtung der Flüssigkeit entspricht der Förderrichtung des Separators oder ist etwas geneigt dazu.

Unter einem selbstfördernder Weitertransport des Güllefeststoff- und/oder des Biogas-Gärsubstratfeststoffes in den stromabwärtigen Behälter ist beispielsweise eine freier Fall der Festphase gemeint.

Zum Beispiel ist es möglich, ausschließlich durch Schwerkraft, also ohne weitere Vorrichtung wie z. B. eine dafür vorzusehende Fördereinrichtung oder ohne ein Pumpe oder dgl. die Festphase vom Auslass in das Innere des Behälters zu überführen.

Beispielsweis besteht genügend Platz zur Unterbringung bzw. Aufnahme eines zweiten Wellenlagers im auslassseitigen Bereich des Gehäuses des Separators.

Beispielsweise können die Weiterförderung der Festphase in den Behälter unterstützende Mittel vorgesehen werden.

Zum Beispiel wird die wesentliche treibende Kraft zur Weiterförderung der Festphase durch die Gravitationskraft bereitgestellt.

Beispielsweise gibt der Auslass eine Auslassrichtung für die weitergeförderte Festphase vor, wobei die Auslassrichtung in einem Winkel gegenüber einer von dem Fördermechanismus vorgegebenen Förderrichtung ausgerichtet ist. Zum Beispiel ist die Auslassrichtung zur Horizontalen geneigt, und senkrecht ausgerichtet oder zusätzlich zur Senkrechten bzw. Vertikalen geneigt. Damit kann der Separator flexibel an unterschiedlichen Positionen relativ zum Behälter positioniert werden. Beispielsweise ist der Separator seitlich neben einer vertikalen Außenwand des Behälters positionierbar. Beispielsweise weist der Separator eine horizontal ausgerichtete Förderrichtung positioniert werden.

Zum Beispiel ist die Auslassrichtung derart, dass die Festphase z. B. unter einem Winkel von 30 bis 60 Winkelgraden z. B. von 45 Winkelgraden zur Senkrechten in das Innere des Behälters gelangt.

Beispielsweise ist der Winkel derart, dass die Auslassrichtung für die weitergeförderte Festphase schräg zur Vertikalen ausgerichtet ist, zum Beispiel um 30 bis 60 Winkelgraden z. B. von 45 Winkelgraden zur Vertikalen geneigt. Beispielsweise gelangt der Güllefeststoff- und/oder Biogas-Gärsubstratfeststoff unter einem Winkel schräg zur Vertikalen in das Innere des Behälters bzw. des Gärbehälters.

Zum Beispiel umfasst die Achse im Bereich des Auslasses Förderflügel. Zum Beispiel unterstützen die Förderflügel den Weitertransport der Festphase bzw. des Fest- oder Dickstoffes aus dem Separator in den Behälter. Zum Beispiel beschleunigen die Förderflügel die Festphase in Richtung des Auslasses bzw. in Richtung einer dazugehörigen Auslassöffnung des Separators.

Beispielsweise ist am Gehäuse im Bereich des Fördermechanismus eine Service-Öffnung ausgebildet. Die Service-Öffnung ist z. B. mit einem Deckel verschließbar. Zum Beispiel ist der Deckel druckstabil und abdichtend an der Service-Öffnung lösbar anbringbar, zum Beispiel mit einer Flanschverschraubung und mit Dichtmitteln wie einer Gummi-Ringdichtung.

Beispielsweise kann im Stillstand des Separators der Deckel abgenommen werden und so das Innere des Separators im Bereich des Fördermechanismus inspiziert werden. Falls nötig kann bei abgenommenem Deckel von außen in das Innere des Separators eingegriffen werden, z. B. um Verkrustungen oder Verstopfungen zu entfernen oder um Beschädigungen festzustellen und zu beheben. Bei abgenommenem Deckel kann z. B. von außen im Bereich der Förderschnecke diese inspiziert werden, z. B. hinsichtlich Verschleiß oder z. B. Anbackungen.

Beispielsweise ist ein Schauglas in dem Deckel der Service-Öffnung integriert vorhanden zum visuellen Einblick von außen ins Innere des Separators. Zum Beispiel weist das Schauglas eine von außen betätigbare Wischlippe auf, die drehbar ist und an der Schauglas-Innenseite anliegt, so dass durch Drehen der Wischlippe die Innenseite des Schauglases von Verschmutzungen befreit werden kann.

Zum Beispiel sind zwei oder mehr identische oder unterschiedliche Service-Öffnungen vorgesehen, zum Beispiel an zwei gegenüberliegenden Stellen am Gehäuse im Bereich des innenliegenden Fördermechanismus. Zum Beispiel ist beidseitig am Gehäuse des Separators jeweils eine Service-Öffnung vorhanden, mit gleicher oder unterschiedlicher Größe der Service-Öffnung.

Zusätzlich zur Service-Öffnung am Gehäuse im Bereich des Fördermechanismus kann zumindest eine weitere Service-Öffnung an anderer Stelle am Gehäuse des Separators vorhanden sein.

Zum Beispiel ist am Gehäuse im Bereich des Auslasses ein Schauglas angeordnet. Damit kann der Bereich des Auslasses während des angetriebenen Betriebs des Separators von außen beobachtet werden bzw. kontrolliert werden. Zum Beispiel dient das Schauglas zur Beobachtung des Förderbetriebs im Trennbetrieb des Separators.

Beispielsweise ist das Schauglas am Gehäuse im Bereich des Güllefeststoff- und/oder Biogas-Gärsubstratfeststoff-Auslasses angeordnet bei einem Gülle- bzw. Gärsubstrat-Separator.

Beispielsweise ist zusätzlich oder alternativ ein Schauglas in einem Deckel integriert, der die Service-Öffnung lösbar abdeckt bzw. verschließt.

Zum Beispiel weist das Schauglas eine von außen betätigbare Wischlippe auf.

Zum Beispiel ist an dem Separator ein multifunktionaler Zugang vorhanden, der ausgebildet ist, dass von außen am Separator z. B. durch eine Service-Person genau eine Funktion oder mehrere unterschiedliche Funktionen erfüllbar sind, z. B. Betriebs- und/oder Instandhaltungsarbeiten auszuführen, z. B. zur Behebung von Betriebsstörungen.

Zum Beispiel ist der multifunktionale Zugang im Bereich des Fördermechanismus, im Bereich des Verdichtermechanismus und/oder im Bereich des Auslasses für die Festphase ausgebildet.

Beispielsweise umfasst der multifunktionale Zugang eine Leitung, die ins Innere des Separators mündet, z. B. mit einer Absperrfunktion wie einem Absperrventil. Beispielsweise umfasst der multifunktionale Zugang einen Anschluss zum Durchlass eines Mediums wie Spülflüssigkeit. Beispielsweise umfasst der multifunktionale Zugang einen Spülanschluss für von außen ins Innere zuführbares Wasser, Presswasser, Substrat oder dergleichen.

Beispielsweise sind an der Achse mehrere Förderflügel befestigt beispielsweise im Bereich des Auslasses und/oder im Bereich des Abwurfes. Die Förderflügel sind z. B. lösbar befestigt an der Achse, z. b. auf einer gemeinsamen Umfangslinie und/oder in axialer Richtung der Achse versetzt zueinander.

Es sind Beispielsweise zwei oder mehr als zwei Förderflügel an der Achse vorhanden zum Beispiel umfänglich regelmäßig versetzt. Beispielsweise sind die Förderflügel im Bereich des Auslasses für die Festphase vorhanden. Beispielsweise sind die Förderflügel an der Achse im Bereich zwischen der Membrane und der auslassseitigen Lagereinrichtung zur Drehlagerung der Achse vorhanden. Zum Beispiel sind die Förderflügel außen an der Achse an einem Abschnitt der Achse vorhanden, der in gerader Verlängerung liegt zu einer zentralen Längsachse eines an den Auslass anschließenden Rohrabschnittes, der in den Behälter mündet. Die von den Förderflügeln radial zur Drehachse der Achse beschleunigte mitgenommene Festphase gelangt damit unmittelbar in Richtung des Inneren des Behälters.

Zum Beispiel ermöglichen die Förderflügel die Bereitstellung von zusätzlichen Förderkräften und einen verbesserten Austrag der Festphase wie des Güllefeststoffs und/oder des Biogas-Gärsubstratfeststoffes.

Beispielsweise ist ein Förderflügel als Abräumer ausgebildet. Zum Beispiel sind die Förderflügel flügelförmig bzw. zungenförmig. Beispielsweise dienen die Förderflügel zur Vermeidung von Verstopfungen und zur Abscheidung der eingedickten Festphase wie des Güllestoff- und/oder des Biogasgärsubstratfeststoffes. Zum Beispiel dienen die Förderflügel der Vermischung von Güllefeststoff und/oder Gärsubstratfeststoff mit eingespültem Substrat wie z. B. Gülle oder Gärsubstrat.

Zum Beispiel ist eine Druckausgleichsleitung zwischen einem Trennbereich-Gehäuseabschnitt des Gehäuses und einem Festphasenbereich-Gehäuseabschnitt des Gehäuses installiert. Damit kann im Falle eines Druckanstiegs im Inneren des Separators, zum Beispiel im Trennbereich und/oder im Festphasenbereich, über ein noch tolerierbares Druckniveau hinaus, vermieden werden, dass Schäden am Separator und dessen Bauteile auftreten. Beispielsweise lassen sich im Trennbetrieb des Separators auftretende Druckspitzen im Trennbereich und/oder im Festphasenbereich vermeiden bzw. minimieren und z. B. auf ein tolerierbares Maß bringen und dauerhaft halten. Damit lässt sich der Trennbetrieb störungsfrei und/oder dauerhaft ohne andernfalls druckspitzenbedingte Unterbrechungen des Betriebs aufrechterhalten.

Beispielsweise gelangt bei einem entsprechenden Druckanstieg in einem Bereich relativ zum anderen Bereich im Separator-Inneren über die Druckausgleichsleitung Material der Festphase in den Bereich mit dem geringeren Druck. Es findet ein Druckabbau im Bereich mit dem höheren Druckniveau statt.

Beispielsweise ist die Druckausgleichsleitung zwischen dem Gehäuseabschnitt, der den Verdichtermechanismus mit der Förderschnecke umgibt, und dem Gehäuseabschnitt, der den Auslassbereich umgibt, vorhanden. Zum Beispiel erstreckt sich die Druckausgleichsleitung am Separator, zum Beispiel außen am Separator, derart, dass ein erstes Ende der Druckausgleichsleitung in den Bereich in Strömungs- bzw. Förderrichtung vor der Membrane mündet und ein zweites bzw. gegenüberliegendes Ende der Druckausgleichsleitung in den Bereich in Strömungs- bzw. Förderrichtung nach der Membrane mündet.

Beispielsweise ist eine Siebtrommel und/oder ein Spaltsieb um die rotierbare Achse in einem Aufnahmerahmen positioniert. Die Siebtrommel und/oder das Spaltsieb sind Teil des Separators.

Beispielsweise sind die Siebtrommel und/oder das Spaltsieb im Bereich der Achse mit der Förderschnecke und radial zur Achse beabstandet vorhanden, zum Beispiel in einem Bereich unterhalb der Achse. Zum Beispiel ist die Siebtrommel und/oder das Spaltsieb in einem unterhalb der Achse mit der Förderschnecke liegenden Abschnitt vorhanden. Eine Wand der Siebtrommel und/oder des Spaltsiebes weist Öffnungen auf, z. B. Löcher und/oder Schlitze. Über die Öffnungen, mit z. B. vorgebbarer Öffnungsgröße je nach Substrat, kann aus dem Substrat abgetrennte Flüssigphase durch Öffnungen einer Wand der Siebtrommel und/oder des Spaltsiebes nach außen treten in Richtung des Flüssigphasen-Auslasses und aus dem Separator abströmen. Feststoff- bzw. Festphasen-Partikel lassen sich durch die Siebtrommel und/oder das Spaltsieb auf der Innenseite bzw. auf der Seite der Achse mit dem Fördermechanismus zurückhalten.

Zum Beispiel sind die Siebtrommel und/oder das Spaltsieb gegen Verdrehung gegenüber dem Gehäuse des Separators gesichert, zum Beispiel gesichert gegen ein Verdrehen um die Längsachse der Achse bzw. Welle. Beispielsweise sind die Siebtrommel und/oder das Spaltsieb schwimmend gelagert.

Es wird ein System zur Erzeugung von Biogas vorgeschlagen, umfassend einen Speicherbehälter zur Speicherung von Gülle und/oder Biogas-Gärsubstrat, einen Gülle- und/oder Biogas-Gärsubstrat-Separator und einen Gärbehälter zur Aufnahme von Güllefeststoff- und/oder Biogas-Gärsubstratfeststoff, wobei der Gülle- und/oder Biogas-Gärsubstrat-Separator nach einer der oben beschriebenen Ausgestaltungen ausgebildet ist und mit dem Gärbehälter gasdicht verbunden ist. Der Gülle- und/oder Biogas-Gärsubstrat-Separator weist einen Auslass für Güllefeststoff- und/oder Biogas-Gärsubstratfeststoff auf, wobei der Güllefeststoff- und/oder Biogas-Gärsubstratfeststoff selbstfördernd in den Gärbehälter gelangt.

Zum Beispiel kann die Gülle Rinder- oder Schweine-Gülle sein. Als ein mögliches Gärsubstrat kommt zum Beispiel Maische in Betracht.

Alternativ zur Nutzung von Gülle- und/oder Biogas-Gärsubstrat im System ist auch jedes andere Substrat, das durch Mikroorganismen abbaubare organische Substanz enthält, nutzbar. Beispielsweise Substrat aus der Landwirtschaft, das zur Biogas- oder Düngerherstellung geeignet ist.

Der Gärbehälter ist beispielsweise als Biogas- oder Vorlagebehälter ausgebildet. Zum Beispiel ist der Gärbehälter nach außen gegen einen Eintritt von Luftsauerstoff abgeschlossen.

Beispielsweise sind zwei oder mehr Separatoren pro Behälter zu dessen Beschickung vorgesehen.

Es wird ein Verfahren zur Separation und Förderung von Gülle- und/oder Biogas-Gärsubstrat vorgeschlagen, umfassend die Schritte:
- Zuführen von Gülle- und/oder Biogas-Gärsubstrat zu einem Gülle- und/oder Biogas-Gärsubstrat-Einlass eines Gülle- und/oder Biogas-Gärsubstrat-Separators,
- Separieren des Gülle- und/oder des Biogas-Gärsubstratfeststoffes aus der Gülle und/oder dem Biogas-Gärsubstrat mittels einem Gülle- und/oder Biogas-Gärsubstrat-Fördermechanismus und einem Verdichtermechanismus. Das Verfahren weist den weiteren Schritt auf:
- gasdichter und selbstfördernder Weitertransport des mit dem Gülle- und/oder Biogas-Gärsubstrat-Separators erzeugten Güllefeststoffs- und/oder Biogas-Gärsubstratfeststoffes zu einem stromabwärts angeordneten Gärbehälter.

Beispielhaft wird das Verfahren mit einem Separator durchgeführt, der nach einer der oben beschriebenen Ausgestaltungen ausgebildet ist. Zum Beispiel ist der Separator ein Gülle- und/oder Biogas-Gärsubstrat-Separator.

Bei dem Verfahren erfolgt ein Betrieb des Gülle- und/oder Biogas-Gärsubstrat-Separators zum Beispiel mittels einer Drucksteuerung. Zum Beispiel weist der Gülle- und/oder Biogas-Gärsubstrat-Separator eine Kontrolleinheit mit einer Rechnereinheit auf, mit welcher der Gülle- und/oder Biogas-Gärsubstrat-Separator betrieben wird. Beispielsweise ist die Kontrolleinheit ausgebildet, mit Sensoren wie Drucksensormittel zusammenzuarbeiten, welche beispielsweise druckbezogene Sensorwerte bezüglich des Gülle- und/oder Biogas-Gärsubstrat-Separators und/oder des Gärbehälters erfasst und der Kontrolleinheit bereitstellt zum Betrieb des Separators.

Beispielsweise wird die auf den Separator bezogene Zuführung des Gülle- oder Biogas-Gärsubstrats überwacht und geregelt über einen Sensor oder mehrere Sensoren, zum Beispiel umfassend einen Druck- und/oder Durchfluss- und/oder Mengen-Messsensor. Der Sensor oder die Sensoren sind beispielsweise am Gehäuse des Separators wie z. B. an einem Zuführgehäuse des Separators montiert. Zum Beispiel wird über einen Drucksensor eine Zuführmenge auf einen abgestimmten Druckwert geregelt, zum Beispiel auf einen Soll-Druckwert.

Zum Beispiel ist der abgestimmte Druckwert abhängig von der jeweiligen Betriebsart des Separators oder des Systems.

Zum Beispiel ist der abgestimmte Druckwert abhängig von der Art der jeweiligen Gülle oder dem jeweiligen Gärsubstrats und/oder von der Art des jeweiligen Güllefeststoffes oder GärsubstratFeststoffes.

Beispielsweise ist ein weiterer Sensor verbaut, der sicherstellt, dass der Gülle- und/oder Biogas-Gärsubstrat-Separator mit einer vorgebbaren Menge von Substrat versorgt wird, zum Beispiel mit genügend Gülle oder Gärsubstrat versorgt wird, zum Beispiel kontinuierlich versorgt wird.

### Figurenbeschreibung

Weitere Merkmale und Vorteile der Erfindung sind anhand der in den Figuren schematisch dargestellten Ausführungsbeispiele näher erläutert. Im Einzelnen zeigt:
Fig. 1 einen Separator in einer Stirnansicht,
Fig. 2 den Separator gemäß Fig. 1 von der Seite in einem Schnitt gemäß der Linie A - A in Fig. 1 in einem ersten Betriebszustand,
Fig. 3 eine Membrane des Separators gemäß Fig. 1 in einer Stirnansicht,
Fig. 4 den Separator gemäß Fig. 2 in einem zweiten Betriebszustand,
Fig. 5 den Separator gemäß Fig. 1 perspektivisch schräg von oben in einem montierten Zustand,
Fig. 6 die Anordnung gemäß Fig. 5 von oben,
Fig. 7 die Anordnung gemäß Fig. 5 von der Seite in einem Schnitt gemäß der Linie B - B in Fig. 6,
Fig. 8 einen Separator in Stirnansicht in einem montierten Zustand und angrenzende Systemteile eines dazugehörigen Systems,
Fig. 9 die Anordnung gemäß Fig. 8 von der Seite in einem Schnitt gemäß der Linie C - C in Fig. 8 und
Fig. 10 die Anordnung gemäß Fig. 9 in einem Schnitt gemäß der Linie D - D in Fig. 9.

Für sich entsprechende Elemente unterschiedlicher Ausführungsbeispiele sind nachfolgend teils die gleichen Bezugszeichen verwendet.

Ein Separator 1 zur Fest-Flüssig-Trennung eines dem Separator 1 zuführbaren Substrats in einem angetriebenen Trennbetrieb des Separators 1. Ein Teil des Substrats durchströmt den Separator 1 im Trennbetrieb in die horizontale Förder- bzw.

### Strömungsrichtung V.

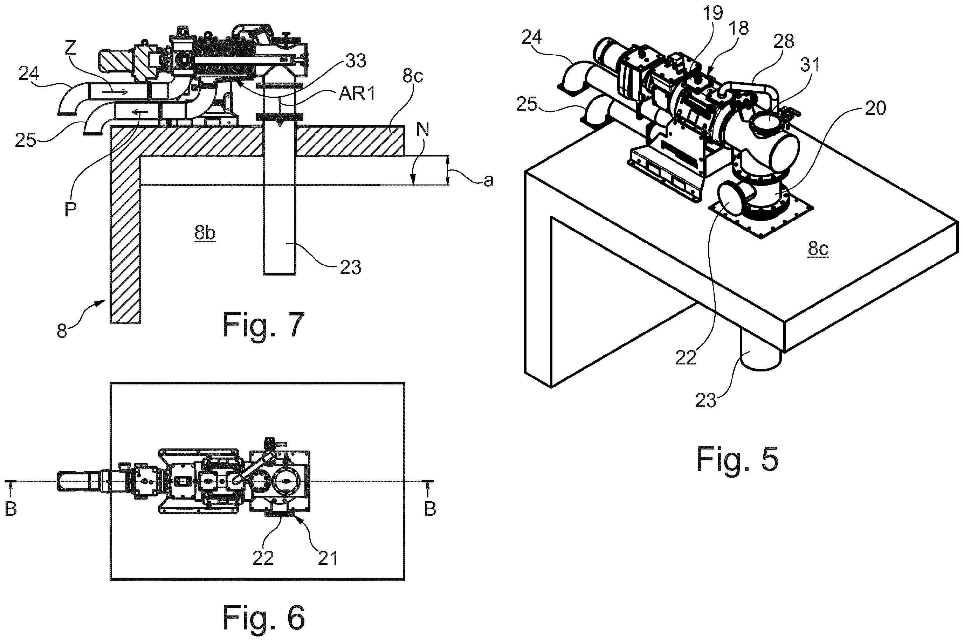

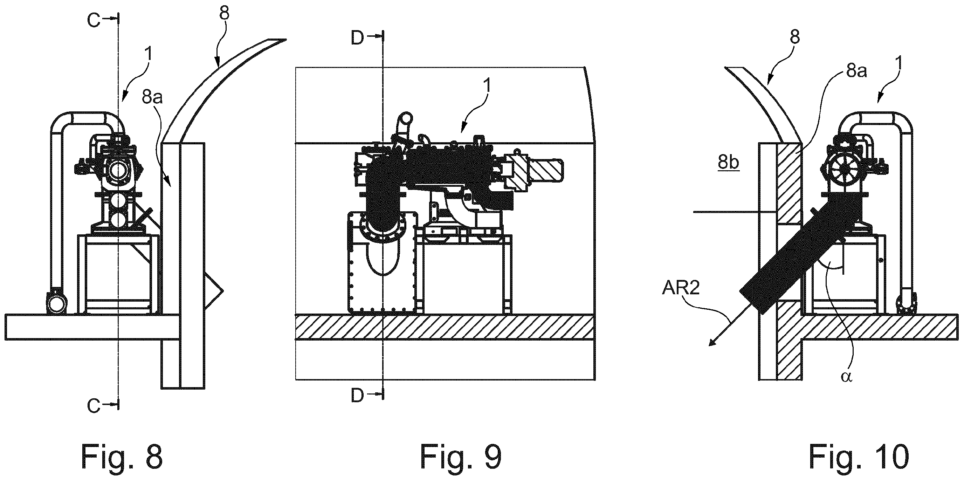

Der Separator 1 weist ein Gehäuse 2, einen Einlass 3 für das Substrat, einen Flüssigphasen-Auslass 4 für eine Flüssigphase, einen Auslass 5 für die Festphase SP, einen Fördermechanismus 6 und/oder einen Verdichtermechanismus 7 für das Substrat auf. Der Auslass 5 ist in einem montierten Zustand des Separators 1 gasdicht mit einem in Strömungsrichtung V stromabwärts angeordneten Behälter 8 verbindbar. Im verbundenen Zustand des Auslasses 5 gelangt die Festphase SP selbstfördernd in den Behälter 8 (s. Fig. 7, 10) bzw. in ein von einer Behälterwand 8a umschlossenes Inneres 8b des Behälters 8.

Der Fördermechanismus 6 umfasst deine Förderschnecke 9, welche auf einer doppelt gelagerten Achse 10 angeordnet ist, zum Beispiel angeschweißt ist. Die Achse 10 ist über die Lagerstellen 26, 27 im Bereich beider Längsenden der Achse 10 beidseitig drehgelagert.

Beispielsweise ist eine Siebtrommel 33 bzw. ein Spaltsieb um die rotierende Achse 10 in einem Aufnahmerahmen im Bereich des Fördermechanismus 6 vorhanden. Durch Sieböffnungen in der Siebtrommel 33 fließt Flüssigphase nach außen und über eine Abführleitung 25 nach außen ab.

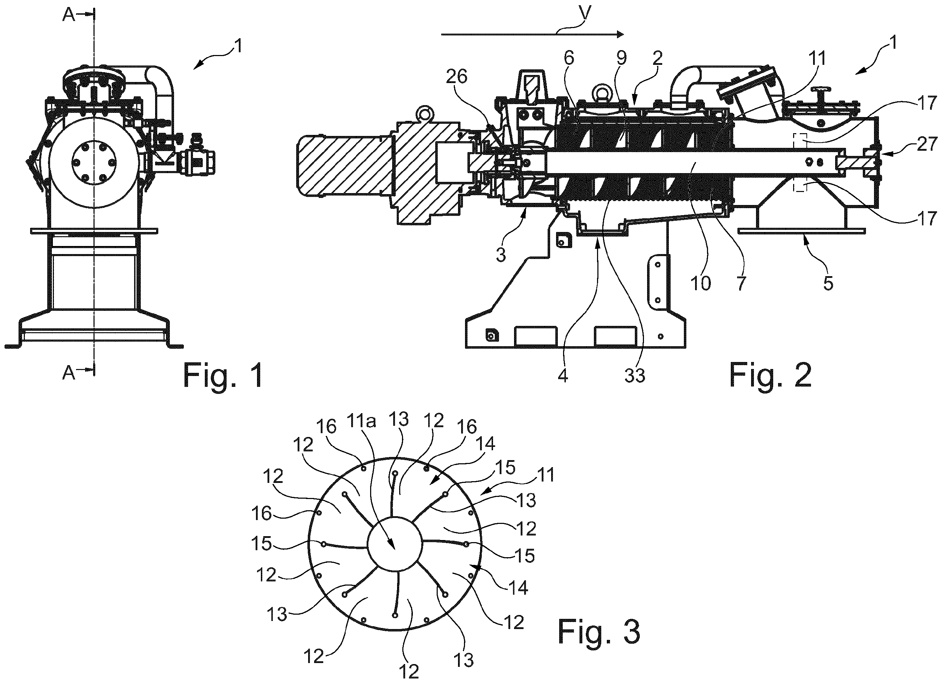

Beispielhaft umfasst der Verdichtermechanismus 7 eine Membrane 11 aus einem elastischen Material. Die ring-scheibenförmige Membrane 11 ist in Fig. 3 in Einzeldarstellung mit einer zentrischen Öffnung 11a gezeigt. Durch die Öffnung 11a ragt die Achse 10 durch. Ein kreisförmiger Außenrand der Membrane 11 ist fest am Gehäuse 2 fixiert, z. B. über Schraubverbindungen, die durch Schraublöcher 16 der Membrane 11 greifen.

Fig. 3 zeigt die unbelastete Membrane 11 bezogen auf einen im Separator 1 montierten Zustand in axialer Ansicht zur Förder- bzw. Strömungsrichtung V. Die Membrane 11 ist in einem nicht belasteten Zustand gezeigt, in welchem elastisch biegbare bzw. ausweichbare Flügelabschnitte 12 der Membrane 11 unbelastet sind. Sämtliche Flügelabschnitte 12 befinden sich gemäß Fig. 2, 3 ausgerichtet in einer von der Membrane 11 im Grundzustand vorgegebenen Ebene. Im vorliegenden Fall gemäß Fig. 3 weist die ringförmige Membrane 11 aus einem Gummimaterial acht identische Flügelabschnitte 12 auf. Jeder Flügelabschnitt 12 ist beidseitig durch jeweils einen Einschnitt 13 in der Membrane 11 freigeschnitten und über ein Filmscharnier 14 mit dem restlichen Teil der Membrane 11 verbunden. Zum Beispiel sind die Einschnitt 13 leicht gebogen in radialer Richtung bzw. im radialen Verlauf. Jeder Einschnitt 13 verläuft von der Öffnung 11a bis zu einer Stelle, die durch ein rundes Loch 15 gebildet ist.

Beispielsweise weist das Loch 15 einen Durchmesser in einer Größenordnung auf, die im Bereich des zwei- oder dreifachen Wertes einer Spaltbreite des Einschnitts 13 liegt. Ein jeweiliges Filmscharnier 14 ist in einem Bereich einer Verbindungslinie zwischen zwei benachbarten Löchern 15 ausgebildet. Je nach einer Dicke und Material der Membrane 11 ist ein Widerstand der Flügelabschnitte 12 gegen eine Ausweichbewegung vorgebbar z. B. abstimmbar auf ein Substrat bzw. dessen Feststoff.

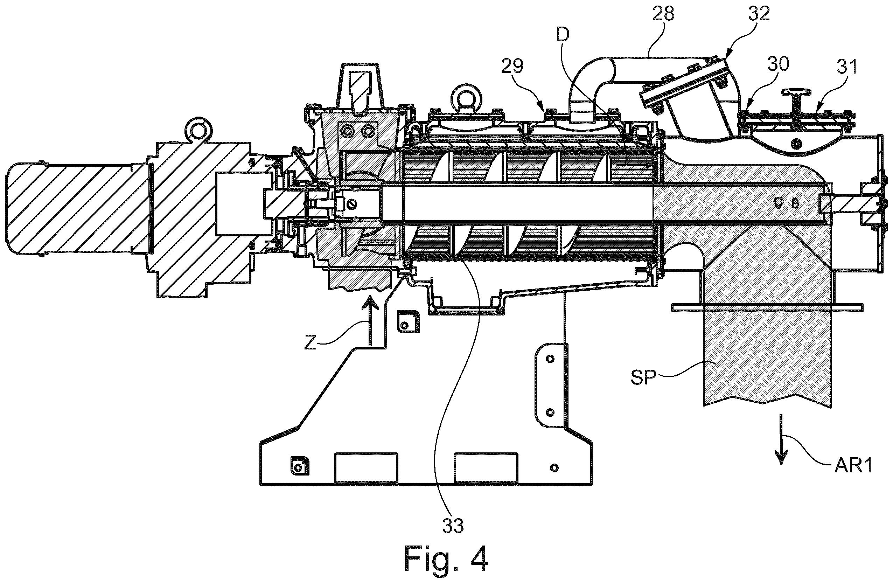

Entlang des zugehörigen Filmscharniers 14 ist jeder Flügelabschnitt 12 elastisch ausweichbar und aus der von der Membrane 11 vorgegebenen Ebene ausweichbar, bei einer ausreichend hohen Druckkraft D (s. Fig. 4), die in axialer Richtung zur Achse 10 aufgrund gestauter Festphase SP auf den jeweiligen Flügelabschnitt 12 wirkt. Die Flügelabschnitte 12 sind selbsttätig rückstellbar aus der ausgelenkten Stellung gemäß Fig. 4 in die nicht ausgelenkte Stellung gemäß Fig. 2 bei axialer bzw. in Förderrichtung V nicht ausreichend hoher Druckkraft D auf die Membrane 11.

Der Auslass 5 gibt eine im Wesentlichen vertikal nach unten weisende Auslassrichtung AR1 vor, so dass die Weiterförderung der Festphase SP ausschließlich durch Gravitationskraft erfolgt (s. Fig. 4, 7). Die Auslassrichtung AR1 steht beispielsweise rechtwinklig zur von dem Fördermechanismus 6 vorgegebenen Förderrichtung V, die in der Regel horizontal ausgerichtet ist.

Gemäß Fig. 2 sind optional vorhandene und gestrichelt angedeutete Förderflügel 17 an der Achse 10 im Bereich des Auslasses 5 vorhanden.

Beispielsweise ist am Gehäuse 2 im Bereich des Fördermechanismus 6 eine Service-Öffnung 18 ausgebildet. Die Service-Öffnung 18 ist im Betrieb des Separators 1 mit einem am Gehäuse 2 anschraubbaren Deckel 19 fest verschlossen (s. Fig. 5).

Eine Revisionsöffnung 21 ist im Bereich eines Zwischenrohres 20 mit einem Flanschdeckel 22 verschließbar vorhanden. Das Zwischenrohr 20 ist in Auslassrichtung AR1 bzw. senkrecht ausgerichtet und mit einem oberen Ende eines ebenfalls senkrechten Tauchrohres 23 verschraubt. Das Tauchrohr 23 reicht abgedichtet durch eine Behälterdecke 8c des Behälters 8. Über ein unteres Ende des Tauchrohres 23 mündet Festphase in das Behälterinnere 8b des Behälters 8, unterhalb eines Oberflächen-Niveaus N der im Behälter 8 gesammelten Festphase. Das Oberflächen-Niveau N liegt gemäß einem vertikalen Abstand a unterhalb einer Innenseite der horizontalen Behälterdecke 8c (s. Fig. 7). In diesem Bereich sammelt sich im Behälter 8 z. B. gebildetes Biogas. Das Biogas ist zur Verwertung aus dem Behälter 8 abführbar.

Gemäß Fig. 7 ist eine Zuführleitung 24 für den Separator 1 ersichtlich, die bis zum Einlass 3 führt und über die z. B. mit einer Druckpumpe Substrat in den Separator 1 gemäß Zuführrichtung Z zugeführt wird. Über die an den Flüssigphasen-Auslass 4 anschließende Abführleitung 25 wird Flüssigphase aus dem Separator 1 in Richtung P abgeführt.

Eine Druckausgleichsleitung 28 ist zwischen einem Trennbereich-Gehäuseabschnitt 29 des Gehäuses 2 und einem Festphasenbereich-Gehäuseabschnitt 30 des Gehäuses 2 installiert.

Zum Beispiel weist der Separator 1 einen Spülanschluss 32 auf. Der Spülanschluss 32 ist z. B. im Festphasenbereich-Gehäuseabschnitt 30 des Gehäuses 2 vorhanden.

Zum Beispiel ist am Gehäuse 2 im Bereich des Auslasses 5 bzw. am Festphasenbereich-Gehäuseabschnitt 30 ein Schauglas 31 angeordnet.

Gemäß der zu Fig. 4-7 alternativen Anordnung gemäß der Fig. 8 bis 10 gibt der Auslass 5 des Separators 1 eine Auslassrichtung AR2 für die weitergeförderte Festphase SP vor, wobei vom Auslass 5 ein gewinkelter Rohrabschnitt anschließt der sich nach unten erstreckt und eine Auslassrichtung AR2 in einem Winkel α gegenüber einer Senkrechten für die Weiterförderung der Festphase SP vorgibt (s. Fig. 10). Die Anordnung gemäß der Fig. 8 bis 10 zeigt einen Auslass 5 mit einem Auswurf der Festphase SP in einem Wandeinbau einer Behälterwand 8a des Behälters 8.

### Bezugszeichenliste

- 1: Separator
- 2: Gehäuse
- 3: Einlass
- 4: Flüssigphasen-Auslass
- 5: Auslass
- 6: Fördermechanismus
- 7: Verdichtermechanismus
- 8: Behälter
- 8a: Behälterwand
- 8b: Behälterinneres
- 8c: Behälterdecke
- 9: Förderschnecke
- 10: Achse
- 11: Membrane
- 11a: Öffnung
- 12: Flügelabschnitt
- 13: Einschnitt
- 14: Filmscharnier
- 15: Loch
- 16: Schraubloch
- 17: Förderflügel
- 18: Service-Öffnung
- 19: Deckel
- 20: Zwischenrohr
- 21: Revisionsöffnung
- 22: Flanschdeckel
- 23: Tauchrohr
- 24: Zuführleitung
- 25: Abführleitung
- 26: Lagerstelle
- 27: Lagerstelle
- 28: Druckausgleichsleitung
- 29: Trennbereich-Gehäuseabschnitt
- 30: Festphasen-Gehäuseabschnitt
- 31: Schauglas
- 32: Spülanschluss
- 33: Siebtrommel

## Patentansprüche

1. Separator (1) zur Fest-Flüssig-Trennung eines dem Separator (1) zuführbaren Substrats in einem angetriebenen Trennbetrieb des Separators (1), wobei im Trennbetrieb ein Teil des Substrats den Separator (1) in eine Strömungsrichtung durchströmt und das Substrat in eine Flüssigphase und in eine Festphase trennbar ist, die aus dem Separator (1) abführbar sind, wobei der Separator (1) ein Gehäuse (2), einen Einlass (3) für das Substrat, einen Flüssigphasen-Auslass (4), einen Auslass (5) für die Festphase, einen Fördermechanismus (6) und/oder einen Verdichtermechanismus (7) für das Substrat umfasst, **dadurch gekennzeichnet, dass** der Auslass (5) gasdicht mit einem in Strömungsrichtung stromabwärts angeordneten Behälter (8) verbindbar ist, wobei im verbundenen Zustand des Auslasses (5) die Festphase selbstfördernd in den Behälter (8) gelangt.

2. Separator (1) nach Anspruch 1, **dadurch gekennzeichnet, dass** der Separator (1) als Gülle- und/oder Biogas-Gärsubstrat-Separator ausgebildet ist, umfassend ein Gehäuse (2), einen Gülle- und/oder Biogas-Gärsubstrat-Einlass (3), einen Güllefeststoff- und/oder Biogas-Gärsubstratfeststoff-Auslass (5), einen Flüssigphasen-Auslass (4), einen Gülle- und/oder Biogas-Gärsubstrat-Fördermechanismus (6) und einen Verdichtermechanismus (7), wobei der Güllefeststoff- und/oder Biogas-Gärsubstratfeststoff-Auslass (5) gasdicht mit einem in Strömungsrichtung stromabwärts angeordneten Gärbehälter (8) verbindbar ist, wobei im verbundenen Zustand des Auslasses (5) der Güllefeststoff und/oder der Biogas-Gärsubstratfeststoff selbstfördernd in den Gärbehälter (8) gelangt.

3. Separator (1) nach Anspruch 1 oder 2, **dadurch gekennzeichnet, dass** der Fördermechanismus (6) eine Förderschnecke (9) umfasst, welche auf einer doppelt gelagerten Achse (10) angeordnet ist.

4. Separator (1) nach einem der vorhergehenden Ansprüche, **dadurch gekennzeichnet, dass** der Verdichtermechanismus (7) eine Membrane (11) aus einem elastischen Material umfasst.

5. Separator (1) nach einem der vorhergehenden Ansprüche, **dadurch gekennzeichnet, dass** der Auslass (5) eine im Wesentlichen vertikal nach unten weisende Auslassrichtung vorgibt, so dass die Weiterförderung der Festphase ausschließlich durch Gravitationskraft erfolgt.

6. Separator (1) nach einem der vorhergehenden Ansprüche, **dadurch gekennzeichnet, dass** der Auslass (5) eine Auslassrichtung für die weitergeförderte Festphase vorgibt, wobei die Auslassrichtung in einem Winkel gegenüber einer von dem Fördermechanismus (6) vorgegebenen Förderrichtung ausgerichtet ist.

7. Separator (1) nach einem der vorhergehenden Ansprüche, **dadurch gekennzeichnet, dass** die Achse (10) im Bereich des Auslasses (5) Förderflügel (17) umfasst.

8. Separator (1) nach einem der vorhergehenden Ansprüche, **dadurch gekennzeichnet, dass** am Gehäuse (2) im Bereich des Fördermechanismus (6) eine Service-Öffnung (18) ausgebildet ist.

9. Separator (1) nach einem der vorhergehenden Ansprüche, **dadurch gekennzeichnet, dass** am Gehäuse (2) im Bereich des Auslasses (5) ein Schauglas (31) angeordnet ist.

10. Separator (1) nach einem der vorhergehenden Ansprüche, **dadurch gekennzeichnet, dass** mehrere Förderflügel (17) an der Achse (10) im Bereich des Auslasses (5) und/oder im Bereich eines Abwurfes für Festphase befestigt sind.

11. Separator (1) nach einem der vorhergehenden Ansprüche, **dadurch gekennzeichnet, dass** eine Druckausgleichsleitung (28) zwischen einem Trennbereich-Gehäuseabschnitt (29) des Gehäuses (2) und einem Festphasenbereich-Gehäuseabschnitt 30) des Gehäuses (2) installiert ist.

12. Separator (1) nach einem der vorhergehenden Ansprüche, **dadurch gekennzeichnet, dass** eine Siebtrommel (33) und/oder ein Spaltsieb um die rotierbare Achse (10) in einem Aufnahmerahmen positioniert ist.

13. System zur Erzeugung von Biogas, umfassend einen Speicherbehälter (8) zur Speicherung von Gülle und/oder Biogas-Gärsubstrat, einen Gülle- und/oder Biogas-Gärsubstrat-Separator (1) und einen Gärbehälter (8) zur Aufnahme von Güllefeststoff- und/oder Biogas-Gärsubstratfeststoff, **dadurch gekennzeichnet, dass** der Gülle- und/oder Biogas-Gärsubstrat-Separator (1) nach einem der vorangehenden Ansprüche ausgebildet und mit dem Gärbehälter gasdicht verbunden ist.

14. Verfahren zur Separation und Förderung von Gülle- und/oder Biogas-Gärsubstrat, umfassend die Schritte:
- Zuführen von Gülle- und/oder Biogas-Gärsubstrat zu einem Gülle- und/oder Biogas- Gärsubstrat-Einlass (3) eines Gülle- und/oder Biogas-Gärsubstrat-Separators (1),
- Separieren des Gülle- und/oder des Biogas-Gärsubstratfeststoffes aus der Gülle und/oder dem Biogas-Gärsubstrat mittels einem Gülle- und/oder Biogas-Gärsubstrat-Fördermechanismus (6) und einem Verdichtermechanismus (7), **gekennzeichnet durch** den weiteren Schritt:
- gasdichter und selbstfördernder Weitertransport des so erzeugten Güllefeststoffs- und/oder Biogas-Gärsubstratfeststoffes zu einem stromabwärts angeordneten Gärbehälter (8).

15. Verfahren nach Anspruch 14, **dadurch gekennzeichnet, dass** der Betrieb des Gülle- und/oder Biogas-Gärsubstrat-Separators (1) mittels einer Drucksteuerung erfolgt.
